# EUROPEAN PATENT APPLICATION

(11) **EP 2 251 025 A1**
(43) Date of publication of application: **17.11.2010**
(21) Application number: 09382069.4
(22) Date of filing: 12.05.2009
(51) Int. Cl.: A61K 38/00, A61K 38/17, A61P 35/00

(54) **Use of inclusion bodies as therapeutic agents**

(71) Applicant: Universitat Autònoma De Barcelona, 08193 Bellaterra, Barcelona (ES); Centro de Investigación Biomédica en Red en Bioingeniería, Biomateriales y Nanomedicina (CIBER BBN), 50018 Zaragoza (ES)
(72) Inventor: García Fruitós, Elena, E-08193, Bellaterra - Barcelona (ES); Vázquez Gómez, Esther, E-08193, Bellaterra - Barcelona (ES); Corchero Nieto, José Luis, E-08193, Bellaterra - Barcelona (ES); Villaverde Corrales, Antonio Pedro, E-08193, Bellaterra - Barcelona (ES)
(74) Representative: ABG Patentes, S.L.

(57) **Abstract**

The invention describes inclusion bodies which, contrarily to the general belief, contain completely active proteins therein. The inclusion bodies will specifically serve as a carrier for the location of therapeutic proteins inside the cell. In addition, the invention also relates to a cell or a pharmaceutical composition comprising said inclusion bodies. Said inclusion bodies formed by therapeutic proteins could be used for the treatment of different diseases, wherein said treatment does not require the generation of an immune response against said therapeutic protein.

## Description

### Field of the Invention

The invention is comprised within the field of disease therapy, specifically in the use of inclusion bodies formed by therapeutic proteins, which can be used for the treatment of different diseases, wherein said treatment does not require the generation of an immune response against said therapeutic protein.

### Background of the Invention

The treatment of several mammalian diseases, such as cancer, diabetes, Parkinson's, etc., requires the administration to the subject of therapeutic proteins which can revert the pathological process involved, and, occasionally, said therapeutic proteins must reach the interior of the cell. There are different ways of targeting these therapeutic proteins into the cell. Although they can be administered in soluble or free form, i.e., without a carrier, in the last few years, alternative strategies are being developed to carry pharmaceutical substances, including peptides, proteins, etc., for the purpose of increasing their stability and internalization. Micelles, liposomes, nanoparticles (e.g., lipid, polymeric, metallic, functionalized nanoparticles, etc.), nanocrystals, cyclodextrins, dendrimers, nanotubes, etc. (Marcato, PD et al., J Nanosci Nanotechnol. 2008 (5): 2216-29) stand out among the strategies developed. Among the drawbacks of said strategies, for example, those based on the use of nanoparticles, their low specificity and the difficulty in producing them easily and quickly are worth mentioning.

In the context of recombinant protein production, there is the general belief that soluble protein species are properly folded and fully functional in contrast to the misfolded and inactive protein versions trapped in insoluble inclusion bodies (IBs) (Baneyx F et al., Nat Biotechnol 2004, 22:1399-1408). Current research no longer supports said hypothesis. The dropping of independent references to inclusion bodies as entities formed by functional protein species with native secondary structure is progressively increasing, and the structural and functional diversity of the model proteins used in these studies does leave little room to speculate about this fact as being an artifact or a peculiarity of a limited number of protein species (Garcia-Fruitós E et al., Microb Cell Fact 2005, 4:27; Jevsevar S et al., Biotechnol Prog 2005, 21:632-639; Garcia-Fruitós E et al., Appl Environ Microbiol 2007, 73:289-294). Recent reviews in this area have shown properly folded proteins included inside IBs (Doglia SM et al., Biotechnol J 2008, 3:193-201; Ventura S et al., Trends Biotechnol 2006, 24:179-185), compromising the paradigm of recombinant protein solubility as equivalent to protein conformational quality (Gonzalez-Montalban N et al., Nat Biotechnol 2007, 25:718-720).

Indeed, the occurrence of functional proteins as important components of bacterial aggregates, as well as the fact that essentially any polypeptide species can be obtained as an IB and that the dimensions of the IBs are comprised between the nanoscale and the microscale (Margreiter G et al., J Biotechnol 2008, 138:67-73), prompts to consider these nanoparticles, spontaneously formed in cell factories, as potential carriers of functional proteins into mammalian cells and organisms, acting as therapeutic nanopills or micropills for protein replacement and other therapies.

For their potential application to be feasible, IBs should be (i) mechanically stable, (ii) non-toxic to mammalian cells, (iii) capable of interacting with and penetrating target cells keeping the biological activity of the forming protein, and (iv) capable of producing the desirable physiological effect associated to the biological activity of the carried protein.

The use of IBs for the production of an oral vaccine against the liver fluke has been described (WO 2004/058816). Said IBs are formed by a cysteine protease of *Fasciola hepatica* fused to the core protein of the hepatitis type B virus (HBV) and are introduced into rats by the oral route, it being observed that they are capable of generating antibodies against said protein. In this case, the IBs act as antigen suppliers for the generation of antibodies by the animal, but they do not act as suppliers of therapeutic proteins for the treatment of diseases which can improve by administering said proteins and do not require the generation of an immune response against said therapeutic proteins.

It is therefore necessary to have alternative systems for supplying proteins of therapeutic interest, in particular for the treatment of diseases which can improve by administering said proteins, which do not require the generation of an immune response against said proteins and which prevent the drawbacks of the systems known in the state of the art; advantageously, said systems should result in an overexpression of the protein of interest with a high yield, without the need for denaturing said protein in order to solubilize it.

### Summary of the Invention

The invention is aimed at the problem of efficiently introducing proteins with therapeutic application inside the cell. The solution provided by the investigators takes advantage of the possibility of overexpressing the therapeutic protein of interest in the form of inclusion bodies (IBs) to introduce said therapeutic protein of interest inside the cell.

It has now been observed that the IBs generated by the aggregation of a recombinant therapeutic protein are not only stable and capable of penetrating the cell, as clearly shown in Example 1 which describes the production and characteristics of IBs formed by the aggregation of the green fluorescent protein (GFP), but said protein recombinant also maintains its biological activity, as illustrated in Example 2, which describes the production of IBs formed by the aggregation of the human Hsp70 chaperone, a potent inhibitor of cell apoptosis. Therefore, said IBs can be used in the treatment of a disease which can improve by administering said therapeutic protein and does not require the generation of an immune response against said therapeutic protein.

Therefore, in a first aspect, the invention relates to an inclusion body (IB) coming from the aggregation of a recombinant therapeutic protein produced in a microorganism or cell line producing inclusion bodies for the treatment of a disease which can improve by administering said therapeutic protein, wherein said treatment does not require the generation of an immune response against said therapeutic protein.

In a particular embodiment, said therapeutic protein is erythropoietin (EPO), corticotropin-releasing hormone (CRH), growth hormone-releasing hormone (GHRH), gonadotropin-releasing hormone (GnRH), thyrotropin-releasing hormone (TRH), prolactin-releasing hormone (PRH), melanotropin-releasing hormone (MRH), prolactin-inhibiting hormone (PIH), somatostatin, adrenocorticotropic hormone (ACTH), somatotropin or growth hormone (GH), luteinizing hormone (LH), follicle-stimulating hormone (FSH), thyrotropin (TSH or thyroid-stimulating hormone), prolactin, oxytocin, antidiuretic hormone (ADH or vasopressin), melatonin, Müllerian inhibiting factor, calcitonin, parathyroid hormone, gastrin, cholecystokinin (CCK), secretin, insulin-like growth factor type I (IGF-I), insulin-like growth factor type II (IGF-II), atrial natriuretic peptide (ANP), human chorionic gonadotropin (hCG), insulin, glucagon, somatostatin, pancreatic polypeptide (PP), leptin, neuropeptide Y, renin, angiotensin I, angiotensin II, factor VIII, factor IX, tissue factor, factor VII, factor X, thrombin, factor V, factor XI, factor XIII, interleukin 1 (IL-1), Tumor Necrosis Factor Alpha (TNF-α), interleukin 6 (IL-6), interleukin 8 (IL-8 and chemokines), interleukin 12 (IL-12), interleukin 16 (IL-16), interferons α, β, gamma, nerve growth factor (NGF), platelet-derived growth factor (PDGF), transforming growth factor β (TGF-β), bone morphogenetic proteins (BMPs), fibroblast growth factors (FGF and KGF), epidermal growth factor (EGF and those related thereto), vascular endothelial growth factor (VEGF), granulocyte colony-stimulating factor (G-CSF), glial growth factor, keratinocyte growth factor, endothelial growth factor, alpha-1 antitrypsin, tumor necrosis factor, granulocyte-macrophage colony-stimulating factor (GM-CSF), cyclosporine, fibrinogen, lactoferrin, tissue-type plasminogen activator (tPA), chymotrypsin, immunoglobins, hirudin, superoxide dismutase, imiglucerase or a chaperone (e.g., Hsp70).

In a particular embodiment, the therapeutic protein which aggregation forms said IB is the human Hsp70 chaperone and the disease which can improve is a disease in which an antiapoptotic effect is required, such as cancer, AIDS, Alzheimer's disease, Parkinson's disease, amyotrophic lateral sclerosis, retinitis pigmentosa, cerebellar degeneration, myelodysplastic syndromes, aplastic anemia, myocardial infarction, apoplexy, etc.

In another aspect, the invention refers to a cell comprising said IB provided by this invention therein.

In another aspect, the invention refers to a pharmaceutical composition comprising a therapeutically effective amount of the IB of the invention and a pharmaceutically acceptable carrier.

Still in another aspect, the invention refers to a pharmaceutical composition comprising a therapeutically effective amount of a cell of the invention and a pharmaceutically acceptable carrier.

### Brief Description of the Drawings

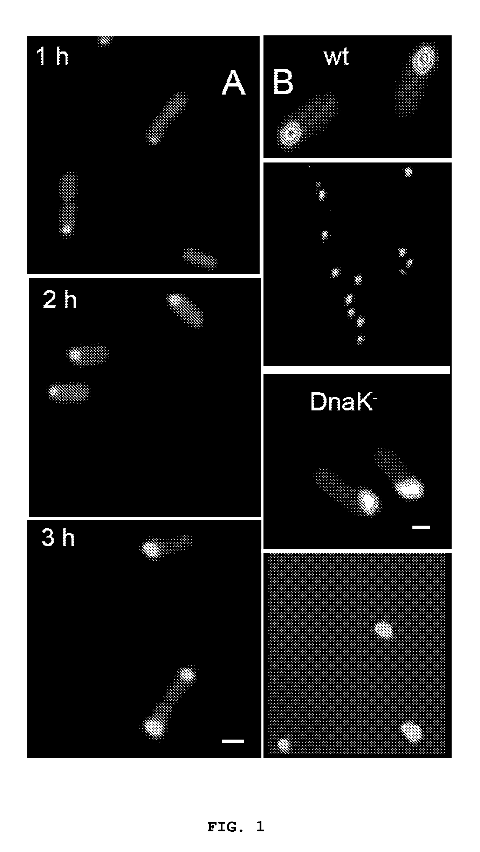

Figure 1 shows the morphological and functional characterization of inclusion bodies (IBs). (A) Confocal microscopy images of wild type (WT) cells overproducing IBs formed by GFP. From top to bottom, cell samples taken at 1 h, 2 h and 3 h after the induction of IB production. (B) Confocal microscopy images using metamorph lookup table of DnaK⁻ and WT cells producing GFP IBs after 2 h of induction of the recombinant gene expression (top), and plain confocal images of IBs purified from these strains after 3h of induction (bottom). With bars in A and B indicate 1 µm.

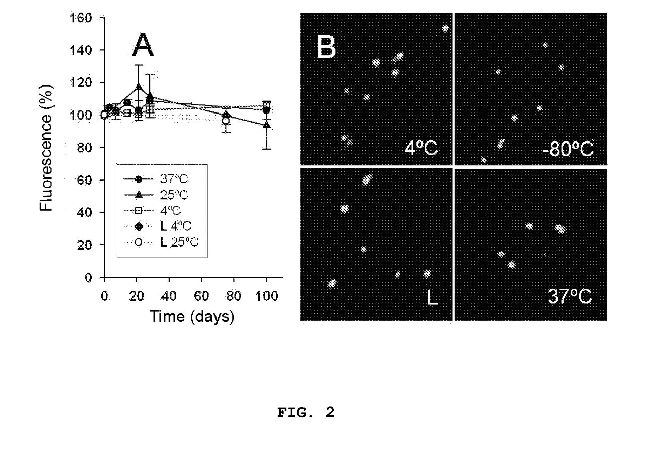

Figure 2 shows the stability of GFP IBs. In (A), stability in aqueous buffer at 37°C, 25°C and 4°C, or lyophilized (L) and further stored at 25°C or 4°C. In (B), confocal microscopy images of purified IBs stored in buffer for one month at 25°C, 4°C and -80°C, and after lyophilization/reconstitution (L).

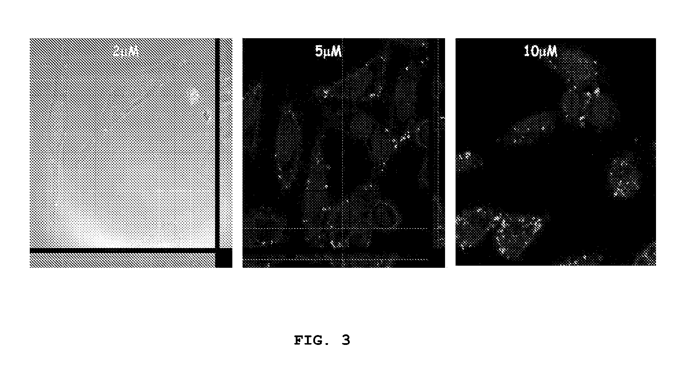

Figure 3 shows the interaction between the IBs and the cell. Optical and confocal images of HeLa cells growing in 35 mm polystyrene plates, 4 h after exposure to different concentrations (indicated on top) of VP1GFP IBs produced in WT cells. Lateral projections in the first two images indicate the internalization of particular IBs as representative examples.

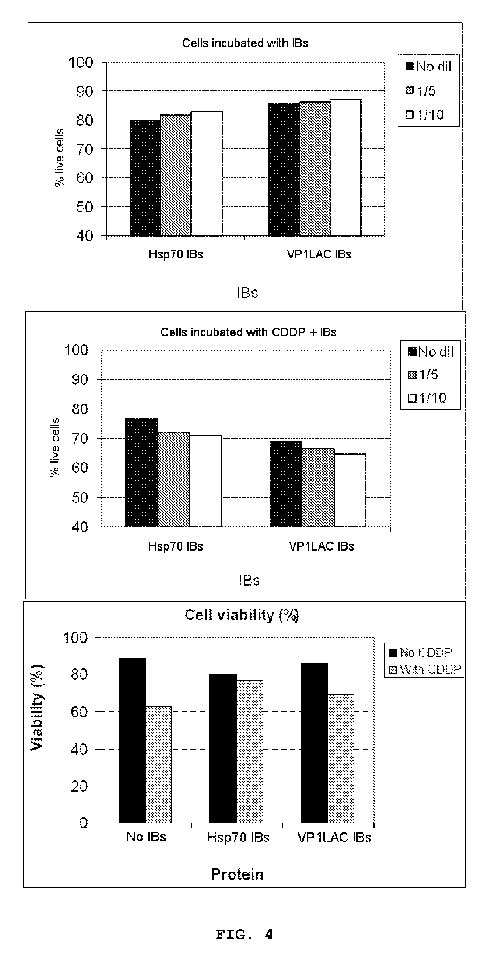

Figure 4 shows the effect of IBs treatment on cisplatin-induced apoptosis in HL-60 cells. (A) Effect of different concentrations of Hsp70 or VP1LAC IBs on cell viability. (B) Effect of undiluted Hsp70 or VP1LAC IBs on cell viability when the cells were simultaneously treated (grey bars) or untreated (black bars) with cisplatin (CDDP). (C) Dose-dependent effect of IBs treatment on CDDP-induced apoptosis.

### Detailed Description of the Invention

The authors of the present invention have identified the possibility of using inclusion bodies (IBs), generated from the production in a host cell of a recombinant therapeutic protein, as therapeutic agents, in particular, in the treatment of a disease which can improve by administering said therapeutic protein and does not require the generation of an immune response against said therapeutic protein.

Therefore, in one aspect, the invention refers to an inclusion body (IB), hereinafter, inclusion body (or IB) of the invention, coming from the aggregation of a recombinant therapeutic protein produced in a microorganism or cell line producing IBs for the treatment of a disease which can improve by administering said therapeutic protein wherein said treatment does not require the generation of an immune response against said therapeutic protein.

As used herein, the expression "inclusion body" or "IB" refers to partial or complete deposits of a recombinant protein in the form of insoluble aggregates, when they are produced in a microorganism or in a suitable recombinant cell system. The abbreviation "IB" will generally be used to refer to an inclusion body (singular) and the abbreviation "IBs" to refer to inclusion bodies (plural). IBs normally have a particle size comprised between 0.05 and 1 µm, although said range can vary and they are refractive to the optical microscope. An IB is normally formed by the aggregation of an insoluble form of the product of a foreign gene. The foreign gene which is introduced into a plasmid could be a gene encoding a heterologous or homologous protein. It is more likely that a heterologous protein forms IB, since is a foreign protein for the cell generating it, but a homologous protein can also produce IB, for example, due to specific sequences of the plasmid promoter increasing the production of said protein. The overexpressed protein is preferably a therapeutic protein.

IBs can accumulate in the cytoplasm or in the periplasm, depending on whether the recombinant protein has been designed to be secreted to the exterior. In order for a protein to be secreted to the exterior, said protein includes a signal sequence, i.e., a peptide sequence capable of promoting the secretion of a protein to the extracellular medium; virtually any signal sequence can be used to put the present invention into practice (e.g., Gallicioti, G. et al. J. Membrane Biology, 183:175-182).

The overexpressed protein can typically represent 70-100% of the IB material, which can contain, in small amounts, other proteins (e.g., membrane proteins, etc.), ribosomal components and a small amount of phospholipids and nucleic acids which are adsorbed after cell lysis. Some chaperones or folding modulators (such as DnaK, GroEL and IbpA/B) are sometimes, but not always, associated with IB formation. As clearly shown in the present invention, IBs are capable of penetrating a cell and being located in a subcellular compartment, wherein the therapeutic protein (the aggregation of which forms the IBs), is active and, therefore, performs its function.

As used herein, the term "therapeutic protein" refers to a protein or polypeptide which exerts a function in its target for stopping or reducing an unwanted physiological change or disorder or promoting a desired biological or therapeutic activity. For the purposes of this invention, beneficial or desired clinical results of the therapeutic protein include, but are not limited to, symptom relief, reduction of the extension of the disease, stabilized pathological state (specifically not worsened), delaying or stopping the progression of the disease, improvement or palliation of the pathological state and remission (both partial and total), both detectable and non-detectable.

Virtually any therapeutic protein capable of forming IBs can be used to put the present invention into practice, for example, hormones, cytokines, coagulation factors, etc. Illustrative non-limiting examples of said therapeutic protein include erythropoietin (EPO), corticotropin-releasing hormone (CRH), growth hormone-releasing hormone (GHRH), gonadotropin-releasing hormone (GnRH), thyrotropin-releasing hormone (TRH), prolactin-releasing hormone (PRH), melanotropin-releasing hormone (MRH), prolactin-inhibiting hormone (PIH), somatostatin, adrenocorticotropic hormone (ACTH), somatotropin or growth hormone (GH), luteinizing hormone (LH), follicle-stimulating hormone (FSH), thyrotropin (TSH or thyroid-stimulating hormone), prolactin, oxytocin, antidiuretic hormone (ADH or vasopressin), melatonin, Müllerian inhibiting factor, calcitonin, parathyroid hormone, gastrin, cholecystokinin (CCK), secretin, insulin-like growth factor type I (IGF-I), insulin-like growth factor type II (IGF-II), atrial natriuretic peptide (ANP), human chorionic gonadotropin (hCG), insulin, glucagon, somatostatin, pancreatic polypeptide (PP), leptin, neuropeptide Y, renin, angiotensin I, angiotensin II, factor VIII, factor IX, tissue factor, factor VII, factor X, thrombin, factor V, factor XI, factor XIII, interleukin 1 (IL-1), Tumor Necrosis Factor Alpha (TNF-α), interleukin 6 (IL-6), interleukin 8 (IL-8 and chemokines), interleukin 12 (IL-12), interleukin 16 (IL-16), interferons α, β, gamma, nerve growth factor (NGF), platelet-derived growth factor (PDGF), transforming growth factor β (TGF-β), bone morphogenetic proteins (BMPs), fibroblast growth factors (FGF and KGF), epidermal growth factor (EGF and those related thereto), vascular endothelial growth factor (VEGF), granulocyte colony-stimulating factor (G-CSF), glial growth factor, keratinocyte growth factor, endothelial growth factor, alpha-1 antitrypsin, tumor necrosis factor, granulocyte-macrophage colony-stimulating factor (GM-CSF), cyclosporine, fibrinogen, lactoferrin, tissue-type plasminogen activator (tPA), chymotrypsin, immunoglobins, hirudin, superoxide dismutase, imiglucerase, chaperones, etc. In a particular embodiment, the therapeutic protein the aggregation of which forms the IBs of the invention is the human Hsp70 chaperone, a potent inhibitor of cell apoptosis.

The IBs of the invention can be obtained by conventional methods which generally comprise introducing the sequence of nucleic acids encoding the therapeutic protein of interest in a suitable expression system which can produce IBs and culturing it under conditions suitable for the production of said IBs.

The person skilled in the art understands that there are different microorganisms and cell lines producing or with the capacity to produce IBs. Illustrative non-limiting examples of said microorganisms and cell lines producing IBs include, although they are not exclusively limited to, bacteria (Parante D et al. (1991), FEBS lett.,77:243), yeasts (Cousens L et al. (1987), Gene, 61:256) and insect cell lines (Thomas CP, et to the (1990). J. Gene. Virol.,71:2073). There is also the possibility that they are generated in mammalian cell lines (Ioannou, Y et al. 1992; Journal of Cell Biology, 119: 1137-50). The introduction of the sequence encoding the therapeutic protein in said microorganisms and cell lines is carried out by means of conventional methods; likewise, the culture media and conditions suitable for the production of said IBs will be chosen; said culture media and conditions are widely known by the persons skilled in the art. The choice of said culture media and conditions will depend on the microorganism or cell line chosen for the production of IBs.

The term "treat" or "treatment" designates a therapeutic treatment, in which the object is to stop (reduce) an unwanted physiological change or disorder, such as, for example, the unwanted cell death. For the purposes of this invention, beneficial or desired clinical results include, but are not limited to, symptom relief, reduction of the extension of the disease, stabilized pathological state (specifically not worsened), delaying or stopping the progression of the disease, improvement or palliation of the pathological state and remission (both partial as total), both detectable as non-detectable, as well as prolonging the survival of the treated subject in comparison with the survival expected if treatment is not received. The subjects in need of treatment include subjects who already suffer from the complaint or disorder and the subjects with a tendency to suffer from the complaint or disorder and the subjects whose complaint or disorder has to be prevented. Said treatment does not require the generation of an immune response against said therapeutic protein.

As used herein, the term "disease which can improve by administering a (said) therapeutic protein" refers to a disease which can be treated with the IBs of the invention, containing a therapeutic protein the administration of which improves the disease to be treated; i.e., beneficial clinical results are obtained by means of administering the suitable IBs of the invention. Illustrative non-limiting examples of said diseases include, although they are not limited to, diseases which can be treated with said protein, including cancer, diabetes, etc, as well as monogenic diseases caused by the lack of a protein, including cystic fibrosis and all the deficiencies treated experimentally with gene therapy. Diseases associated with an increase of apoptosis, i.e., with a reduction of cell proliferation, such as AIDS, etc.; neurodegenerative diseases, e.g., Alzheimer's disease, Parkinson's disease, amyotrophic lateral sclerosis, retinitis pigmentosa, cerebellar degeneration, etc.; myelodysplastic syndromes, e.g., aplastic anemia, etc.; ischemic injury, e.g., myocardial infarction, apoplexy, etc.; reperfusion injury; liver damage, e.g., liver damage caused by alcohol, etc could also be treated.

The IBs of the invention, if desired, can be introduced in cells. Therefore, in another aspect, the invention refers to a cell, hereinafter cell of the invention, comprising an inclusion body of the invention therein. Thus, said cells of the invention include not only the microorganism or cell line producing the IBs of the invention but also target cells internalizing the IBs of the invention. Said cell of the invention can be a prokaryotic or eukaryotic cell. In the case of IBs included inside eukaryotic cells, they could be administered to a subject in need of treatment with autologous cells of said subject containing inclusion bodies of the invention with the suitable therapeutic protein therein, which could be in contact with the IBs of the invention and could subsequently be reimplanted in the subject

In another aspect, the invention refers to a pharmaceutical composition comprising a therapeutically effective amount of an inclusion body of the invention and a pharmaceutically acceptable carrier for the treatment of diseases which do not require the generation of an immune response against the therapeutic protein forming the IB of the invention.

In another aspect, the invention refers to a pharmaceutical composition comprising a therapeutically effective amount of a cell of the invention and a pharmaceutically acceptable carrier for the treatment of diseases which do not require the generation of an immune response against the therapeutic protein forming the IB of the invention.

Examples of diseases which can be treated with the pharmaceutical composition of the invention have been previously mentioned in the present specification.

In the context of the present invention, "therapeutically effective amount" means the amount of inclusion bodies of the invention or of cells of the invention necessary for achieving the desired effect which, in this particular case, is the treatment of diseases which can be treated with the therapeutic protein contained in the IBs of the invention. The therapeutically effective amount of the IB of the invention or of the cell of the invention to be administered will generally depend, among other factors, on the individual who is to be treated, on the severity of the disease that said individual suffers from, on the form of administration chosen, etc.

The pharmaceutical composition of the invention can be administered by any suitable route of administration, for example, oral, by inhalation, parenteral (for example, subcutaneous, intraperitoneal, intravenous, intramuscular route, etc.), rectal route, etc.

Illustrative examples of dosage forms for administration by the oral route include tablets, capsules, granulates, solutions, suspensions, etc., and can contain the conventional excipients, such as binders, diluents, disintegrants, lubricants, wetting agents, etc., and can be prepared by conventional methods. The pharmaceutical compositions can also be adapted for their parenteral administration, in the form of, for example, sterile solutions, suspensions or lyophilized products, in the suitable dosage form; in this case, said pharmaceutical compositions will include the suitable excipients, such as buffers, surfactants, etc. In any case, the excipients will be chosen according to the selected pharmaceutical dosage form.

A review of the different dosage forms for the administration of drugs and their preparation can be found in the book "Tratado de Farmacia Galénica", by C. Faulí i Trillo, 10 Edition, 1993, Luzán 5, S.A. de Ediciones.

In another aspect, the invention relates to a method of treatment of a disease which can be improved by a therapeutic protein comprising administering to a subject in need of treatment a therapeutically effective amount of IBs of the invention, wherein said treatment does not require the generation of an immune response against said therapeutic protein.

In another aspect, the invention relates to a method of treatment of a disease which can be improved by a therapeutic protein, wherein said treatment does not require the generation of an immune response against said therapeutic protein, comprising administering to a subject in need of treatment autologous cells of said subject containing inclusion bodies of the invention with the suitable therapeutic protein therein. Briefly, to put said method of treatment into practice, cells are extracted from the subject to be treated, they are contacted with IBs of the invention containing the suitable therapeutic protein under conditions which allow internalizing said IBs of the invention in said cells, and, subsequently, they are implanted by conventional methods in the subject to be treated. The therapeutic protein present in the IBs of the invention can produce the desired effect by different mechanisms, for example, replacing a deficient protein, or exerting their effect on other protein or on said cells, etc. The IBs of the invention can likewise be used for an *in vivo* method of treatment, wherein the IB of the invention would be administered to a patient with a disease which can be treated with said IBs.

The following examples illustrate the invention and must not be considered as limiting the scope thereof.

### EXAMPLE 1

### Production and characterization of IBs by aggregation of GFP

This assay was performed to analyze if IBs produced in bacteria could be internalized by eukaryotic cells and if said internalization could inactivate the biological activity of the protein. To carry out this example IBs were produced based on the green fluorescent protein (GFP).

### MATERIALS AND METHODS

### 1.1 Production of inclusion bodies

Inclusion bodies (IBs) were produced in *Escherichia coli* MC4100 strains (WT regarding protein folding and degradation, araD139 Δ(argF-lac) U169 rpsL150 relA1 flbB5301 deoC1 ptsF25 rbsR) and in a strain derived thereof, JGT20 (deficient in the main chaperone DnaK, dnak756 thr::Tn10), hereinafter DnaK⁻ strain. These strains were transformed with the expression vector pTVPIGFP (ApR) (Garcia-Fruitós E et al., Microb Cell Fact 2005, 4: 27), encoding the green fluorescent protein (GFP) fused at the amino terminus to VP1, the pentamer-forming capsid protein of Foot and Mouth Disease Virus (FMDV) (Gonzalez-Montalban N et al., Biochem Biophys Res Commun 2007, 355:637-642) 18]. This viral protein, being highly hydrophobic, directs the deposition of fusion proteins as inclusion bodies (Doglia SM et al., Biotechnol. J 2008, 3:193-201). A similar construct, VP1LAC, encodes a previously described beta-galactosidase fusion (Garcia-Fruitós E et al., Microb Cell Fact 2005, 4: 27). The recombinant genes were expressed under the control of an isopropyl beta-D-1-thiogalactopyranoside (IPTG) inducible-trc promoter. The bacteria were cultured in Luria Bertani (LB) rich medium (Sigma-Aldrich, 28760 Madrid, Spain), supplemented with 100 µg/ml of ampicillin, and the recombinant gene expression was induced by adding 1 mM IPTG. IBs were clearly detectable after 1 h of IPTG addition.

### 1.2 Purification of the inclusion bodies (IBs)

Samples of 200 ml of bacterial cultures were centrifuged at 4°C at 5.000 g for 5 minutes and resuspended in 50 ml of lysis buffer (50 mM TrisHC1 pH 8.1, 100 mM NaCl and 1 mM EDTA). Ice-jacketed samples were sonicated for 25 to 40 minutes, at 40% of amplitude under 0.5 s cycles. Once sonicated, 28 µl of 100 mM phenylmethanesulphonylfluoride (PMSF) and 23 µl of lysozime were added to samples that were incubated at 37°C under agitation for 45 min. After that, 40 µl of Nonidet P40 (NP-40) were added and the mixture was kept for 1h at 4°C under agitation. DNA was removed with 120 µl of 1 mg/ml DNase and 120 µl of 1 M Mg₂SO₄ for 45 min at 37°C under agitation. Finally, samples were centrifuged at 4°C at 15000 g for 15 min and the pellet, containing pure IBs, was washed with lysis buffer containing 0.5% Triton X-100 and stored at -20°C until analysis.

### 1.3 Microscopic analysis of the bacteria and the IBs

Samples were analyzed by using a Leica TSC SP2 AOBS confocal fluorescence microscope (Leica Microsystems Heidelberg GmbH, Manheim, Germany) after excitation at 488 nm, and images were recorded at emission wavelengths between 500 and 600 nm (63X (NA 1.4 oil) using a Plan Apochromat objective (zoom 8; 1,024 by 1,024 pixels). For the analysis of bacterial cells producing fluorescent IBs, samples taken 1, 2 or 3h after IPTG induction were fixed with 0.2% formaldehyde in phosphate buffered saline (PBS) and stored at 4°C until their use. Isolated IBs were resuspended in 20 ml of PBS.

### 1.4 Stability analyses

IBs obtained in DnaK⁻ cells for 5 h were diluted in PBS with 10 g/l bovine serum albumin (BSA) and 60 g/l sucrose, in the presence of gentamicin at 40 mg/l, penicillin at 100 U/ml and streptomycin at 10 µg/ml, and aliquots incubated at different temperatures (37°C, 25°C or 4°C). At different times, samples were frozen at -80°C until fluorescence determination. Fluorescence was analyzed in a Cary Eclipse fluorescence spectrophotometer (Variant, Inc., Palo Alto, CA) by using an excitation wavelength of 450 nm and detecting the fluorescence emission at 510 nm. Results are referred to as the percentage of remaining activity or fluorescence with respect to control samples kept at -80°C, that were fully stable. Another set of samples was lyophilized in a Cryodos-80 lyophilizer, from Telstar and stored at either 4°C or 25°C until analysis.

### 1.5 Confocal laser scanning microscopy

HeLa and NIH3T3 cell cultures were seeded at a density of 70% on glass plates (MatTek Corporation, Ashland, MA, USA) 24 h before adding VP1GFP inclusion bodies at different concentrations: 2 µM, 5 µM and 10 µM. Four hours after adding the IBs, the living cells were examined using a spectral confocal Leica TCS SP5 AOBS (Leica Microsystems, Mannheim, Germany) using a Plan Apochromat lens (63X, N.A. 1.4 oil). For nuclear and plasma membrane labeling, cells were incubated with 5 µg/ml of Hoechst 33342 and 5 µg/ml of CellMask (both from Molecular Probes, Inc., Eugene, OR, USA ) respectively for 5 min at room temperature, and washed twice prior to confocal detection. Nuclei were excited with 405 nm diode laser beam, and detected at 414-461 nm (blue channel); plasma membrane was detected by exciting with the light of a 633 nm helium neon laser and fluorescence was detected at 656-789 nm (far red channel); finally, Argon laser 488-nm line was used for imaging VP1GFP IBs (green channel, emission = 500-537 nm).

### RESULTS

GFP-based inclusion bodies (IBs) have been proved to be excellent models to verify the conformational status and biological activity of the embedded proteins (Garcia-Fruitós E et al. Microb Cell Fact 2005, 4:27; Garcia-Fruitós E et al. Appl Environ Microbiol 2007, 73:289-294). Therefore, the inventors chose this model to evaluate the mechanical and functional stability of bacterial IBs, during and after purification. Figure 1A shows the growth of these particles in bacteria producing a recombinant GFP (VP1GFP), showing a defined growth pattern, with a regular volumetric growth is shown. Figure 1B shows microscopy confocal images indicating the location of the fluorescence of IBs produced in WT bacteria or in mutants lacking the chaperone DnaK, in which IBs are larger, and the corresponding confocal images of IBs after purification (bottom of each panel), indicating a high mechanical stability of IBs exerted by the mechanical forces acting during purification, including ultrasonic disruption of the cells and centrifugation are shown.

To further confirm the stability of IBs, especially regarding the biological activity of the protein included therein, fluorescence of VP1GFP IBs was monitored in different storage conditions, including freezing and lyophilization, which are commonly used for biological material. As observed in Figure 2A, IBs were fully functionally stable during long time periods even at 37°C, making possible their potential use under physiological conditions, both in cell cultures and in whole organisms, since the temperature did not compromise biological activity. Furthermore, the morphology of IBs was unchanged under all these conditions (Figure 2B), indicative again of a robust mechanical stability.

At this stage, it was decided to check how IBs could interact with cultured mammalian cells and especially, if this interaction could inactivate the biological activity of the protein, monitored by the fluorescence of an VP1GFP IB, since neither the stability nor the biological activity of IBs during such interaction had ever been analyzed. Figure 3 shows cultured HeLa cells 4 h after exposure at different concentrations of IBs (2, 5 and 10 µM), analyzed under conventional optical microscopy (2 µM) or confocal microscopy with cell staining (5 and 10 µM). As can be observed, at these doses, cells were completely healthy showing no cytopathic effects, while IBs appeared as intimately interacting with cell membranes, and some of them clearly internalized into the cell cytoplasm (see the lateral projections at 2 and 5 µM). These findings strongly support the concept that bacterial IBs bind and penetrate mammalian cells, keeping the conformational status and biological activity of the forming protein and maintaining their mechanical stability.

### EXAMPLE 2

### Production and characterization of IBs by the aggregation of Hsp70

In view of the data obtained in Example 1, the inventors investigated if a therapeutic protein carried by IBs could show biological effects in the cells exposed to IBs. To that end, the human Hsp70 chaperone, a potent inhibitor of cell apoptosis (Garrido C et al. Cell Cycle 2003, 2: 579-584), among other activities of therapeutic value (Calderwood SK et al. Eur J Immunol 2005, 35: 2518-2527), was chosen as a model protein.

### MATERIALS AND METHODS

### 2.1 Production of inclusion bodies

Following a protocol such as that described in section 1.1 of Example 1, duly adapted, inclusion bodies (IBs) were produced in strains of *E. coli* BL21 (DE3) transformed with the commercial expression vector EX-Q0172-B01 (OmicsLink ORF Expression Clone, of GeneCopoeia), expressing the human Hsp70 protein with a histidine tag fused at the N-terminus. The bacterial cells were cultured in LB rich medium supplemented with 100 µg/ml of ampicillin, and the recombinant gene expression was induced by adding 1 mM IPTG. The IBs were clearly detectable 1 h after adding IPTG.

The IBs formed by the aggregation of Hsp70 were purified following a procedure such as that described in section 1.2 of Example 1.

### 2.2 Apoptosis assay

Cell apoptosis was determined by a flow cytometric assay with Annexin V-FITC57 by using an Annexin V-FITC Apoptosis Detection Kit (Roche). Exponential growth of the HL-60 cells (acute promielocytic leukemia, ATTCC no. CLL-240) was adjusted to 3×10⁵ cells/ml, seeded in six-well plates at 2.5 ml/well. Cells were exposed to the IC50 concentration of cisplatin (15.6 µM). Simultaneously to the cisplatin addition, 100 µl of different dilutions of inclusion bodies solutions were added to each well. As controls, the same amount of inclusion bodies were added to cells in the absence of cisplatin, for the purpose of detecting putative deleterious effect of IBs on cells. After 24 h of incubation, cells were subjected to staining with the Annexin VFITC and propidium iodide, as recommended by the manufacturer. The amount of apoptotic cells was determined by flow cytometry (FACSCalibur, BD Biosciences, San José, CA).

### RESULTS

The inventors decided to analyze if the cells exposed to apoptotic agents such as cisplatin and treated subsequently with Hsp70 IBs could maintain their viability, upon being induced by the protein contained in the IBs. Figure 4 shows, in the first place, the potential cytotoxic effects of Hsp70 IBs and also of VP1LAC (Example 1) used as negative controls in the subsequent experiments, are first shown. As observed in Figure 4A, cell growth was unaffected by the exposure of different types of IBs as presumed from direct visualization (Figure 3). When cisplatin was added to the cultures, cell viability mediated by apoptosis was significantly reduced (Figure 4B), and the same event was observed when cells were simultaneously exposed to VP1LAC IBs. However, in the presence of Hsp70 IBs, apoptotic events conducing to cell death were not evident, proving that the human Hsp70 chaperone contained in IBs was able to perform its natural biological activities. This cytoprotective effect was clearly dependent on the used concentration of IBs (Figure 4C). All these data prove that proteins produced as IBs can be administered to cells to produce a therapeutic effect associated to their biological activities, and point out that IBs are promising nanoparticles with therapeutic value, which in addition, are mechanically and functionally stable, fully biocompatible and easily producible in microbial cell factories.

## Claims

1. An inclusion body coming from the aggregation of a recombinant therapeutic protein produced in a microorganism or cell line producing inclusion bodies for the treatment of a disease which can improve by administering said therapeutic protein, wherein said treatment does not require the generation of an immune response against said therapeutic protein.

2. The inclusion body according to claim 1, wherein the therapeutic protein is selected from the group consisting of erythropoietin (EPO), corticotropin-releasing hormone (CRH), growth hormone-releasing hormone (GHRH), gonadotropin-releasing hormone (GnRH), thyrotropin-releasing hormone (TRH), prolactin-releasing hormone (PRH), melanotropin-releasing hormone (MRH), prolactin-inhibiting hormone (PIH), somatostatin, adrenocorticotropic hormone (ACTH), somatotropin or growth hormone (GH), luteinizing hormone (LH), follicle-stimulating hormone (FSH), thyrotropin (TSH or thyroid-stimulating hormone), prolactin, oxytocin, antidiuretic hormone (ADH or vasopressin), melatonin, Müllerian inhibiting factor, calcitonin, parathyroid hormone, gastrin, cholecystokinin (CCK), secretin, insulin-like growth factor type I (IGF-I), insulin-like growth factor type II (IGF-II), atrial natriuretic peptide (ANP), human chorionic gonadotropin (hCG), insulin, glucagon, somatostatin, pancreatic polypeptide (PP), leptin, neuropeptide Y, renin, angiotensin I, angiotensin II, factor VIII, factor IX, tissue factor, factor VII, factor X, thrombin, factor V, factor XI, factor XIII, interleukin 1 (IL-1), Tumor Necrosis Factor Alpha (TNF-α), interleukin 6 (IL-6), interleukin 8 (IL-8 and chemokines), interleukin 12 (IL-12), interleukin 16 (IL-16), interferons α, β, gamma, nerve growth factor (NGF), platelet-derived growth factor (PDGF), transforming growth factor β (TGF-β), bone morphogenetic proteins (BMPs), fibroblast growth factors (FGF and KGF), epidermal growth factor (EGF and those related thereto), vascular endothelial growth factor (VEGF), granulocyte colony-stimulating factor (G-CSF), glial growth factor, keratinocyte growth factor, endothelial growth factor, alpha-1 antitrypsin, tumor necrosis factor, granulocyte-macrophage colony-stimulating factor (GM-CSF), cyclosporine, fibrinogen, lactoferrin, tissue-type plasminogen activator (tPA), chymotrypsin, immunoglobins, hirudin, superoxide dismutase, imiglucerase and chaperones.

3. The inclusion body according to claim 1, wherein the therapeutic protein is the human Hsp70 chaperone and the disease which can be improved is a disease in which an antiapoptotic effect is required.

4. A cell comprising an inclusion body therein according to any of claims 1 to 3.

5. A pharmaceutical composition comprising a therapeutically effective amount of the inclusion body according to any of claims 1 to 3 and a pharmaceutically acceptable carrier.

6. A pharmaceutical composition comprising a therapeutically effective amount of the cell according to claim 4 and a pharmaceutically acceptable carrier.
